Europäisches Patentamt

European Patent Office

Office européen des brevets

(19)

(11) Numéro de publication : **0 399 903 B1**

(12) **FASCICULE DE BREVET EUROPEEN**

(45) Date de publication du fascicule du brevet :
23.12.92 Bulletin 92/52

(51) Int. Cl.⁵ : **A61K 31/645,** A61K 9/18,
A61K 47/48

(21) Numéro de dépôt : 90401370.3

(22) Date de dépôt : 22.05.90

(54) **Nouvelles compositions à base d'imipramine.**

(30) Priorité : 24.05.89 FR 8906781

(43) Date de publication de la demande :
28.11.90 Bulletin 90/48

(45) Mention de la délivrance du brevet :
23.12.92 Bulletin 92/52

(84) Etats contractants désignés :
AT BE CH DE DK ES FR GB GR IT LI LU NL SE

(56) Documents cités :
STN INTERNATIONAL INFORMATION SERVI-
CES, BASE DE DONNEES: CHEMICAL ABS-
TRACTS, numéro d'accès 111194557, vol. 111,
no. 21,résumé no. 194557r, Columbus, Ohio,
US; T. HOSHINO et al.: "Change in photochemical reaction of clomipramine by inclusioncomplexations of .beta.- and
dimethyl-.beta.-cyclodextrins", & YAKUGAKU
ZASSHI, 109(2), 107-12, 1989
STN INTERNATIONAL INFORMATION SERVI-
CES, BASE DE DONNEES: CHEMICAL ABS-
TRACTS, numéro d'accès 108118829, vol. 108,
no. 14,résumé no. 118829d, Columbus, Ohio,
US; C. BARBERO et al.: "Thermodynamic
study of interactions between cycloheptaamylose anddibenzazepines in aqueous solution", & AN. QUIM., SER. A, 83(3), 285-9, 1987

(73) Titulaire : **RHONE-POULENC SANTE
20, avenue Raymond Aron
F-92160 Antony (FR)**

(72) Inventeur : **Coutel-Egros, Anne
3 avenue de la Résidence
F-92160 Antony (FR)**

(74) Mandataire : **Le Pennec, Magali et al
RHONE-POULENC SANTE Service Brevets
Santé, 20 Avenue Raymond Aron
F-92160 Antony (FR)**

Il est rappelé que : Dans un délai de neuf mois à compter de la date de publication de la mention de la
délivrance du brevet européen toute personne peut faire opposition au brevet européen délivré, auprès
de l'Office européen des brevets. L'opposition doit être formée par écrit et motivée. Elle n'est réputée
formée qu'après paiement de la taxe d'opposition (Art. 99(1) Convention sur le brevet européen).

## Description

La présente invention concerne une nouvelle composition pharmaceutique à base d'imipramine ou d'un de ses dérivés. Elle concerne plus particulièrement une nouvelle composition pharmaceutique solide destinée à une administration par voie orale de l'imipramine ou d'un de ses dérivés.

L'imipramine est commercialisée actuellement sous la marque TOFRANIL sous forme de comprimés ou de solution à base de chlorhydrate. La trimipramine qui est un dérivé de l'imipramine est commercialisée sous la marque SURMONTIL sous forme de comprimés ou de solution, les comprimés sont constitués de maléate de trimipramine alors que les solutions sont constituées de méthane sulfonate.

Les solutions aqueuses d'imipramine ou de trimipramine présentent deux inconvénients: d'une part elles ont une très forte amertume qui rend leur administration par voie orale difficilement acceptable surtout pour les enfants et les personnes âgées, d'autre part la division des formes aqueuses est toujours un problème, en traitement ambulatoire, surtout pour des personnes âgées. Ces solutions présentent en plus un phénomène étonnant, lorsqu'elles sont évaporées à sec le produit obtenu, initialement cristallisé, se présente sous forme d'une gomme dont la remise en solution est impossible et qui ne permet plus aucune mise en forme pharmaceutique. La nature du produit de transformation après mise en solution aqueuse est inconnue à ce jour.

Les deux inconvénients précités c'est à dire l'amertume des solutions aqueuses et la formation de gomme qui n'ont aucun lien entre eux ont pu être résolus par la même solution. Cette solution consiste à inclure l'imipramine ou ses dérivés et ses sels dans la cyclodextrine.

Cette inclusion nécessite au moins deux moles de cyclodextrine par mole d'imipramine, de ses dérivés ou de ses sels. On préfère employer entre deux et quinze moles de cyclodextrine par mole d'imipramine, de ses dérivés ou de ses sels.

Parmi les dérivés de l'imipramine on peut citer la trimipramine et parmi ses sels on peut citer le chlorhydrate, le méthane sulfonate. La cyclodextrine utilisée est choisie parmi l'α-cyclodextrine, la β-cyclodextrine et la γ-cyclodextrine. On préfère utiliser la β-cyclodextrine.

Le procédé d'inclusion dans la cyclodextrine de l'imipramine ou de ses dérivés et de ses sels consiste à mettre en solution l'imipramine, son dérivé ou son sel et la cyclodextrine dans une faible quantité d'eau ou de solvant, à mélanger soigneusement le mélange obtenu et à évaporer ledit mélange. Cette évaporation peut être réalisée indifféremment par lyophilisation, sans nécessité d'adjoindre ni diluant ni liant, ou par séchage par tout moyen tel que par exemple l'étuve.

La composition pulvérulente obtenue après évaporation peut subir une mise en forme pharmaceutique tel que la mise en sachets, la compression ou la granulation. Il est toujours possible avant la mise en forme définitive d'ajouter des édulcorants, des aromatisants, des agents sucrants, des conservateurs et des agents colorants. Dans le cadre de la présente invention nous préférons une mise en forme en sachets prêts à l'emploi qui présentent les avantages d'une prise orale facile surtout chez l'enfant et une distribution unitaire avantageuse pour l'enfant comme pour les personnes âgées.

Ces compositions après remise en solution ont un goût insipide et une présentation tout à fait acceptable pour le consommateur.

La présente invention sera plus complètement décrite à l'aide des exemples suivants

## EXEMPLE 1

On met en oeuvre les compositions suivantes:

```
trimipramine méthane sulfonate : 6,64 g        3,38 g           1,66 g

ß-cyclodextrine                   50,00 g       50,00 g          50,00 g

eau                               26,90 g       26,71 g          26,80 g

                    cyclodextrine
rapport molaire  ------------        2,6           5,2             10,4
                    trimipramine
```

On dissout la trimipramine méthane sulfonate dans l'eau, on mouille la cyclodextrine avec la solution précédente. On mélange sous agitation magnétique pendant deux heures à température ambiante. On divise le mélange dans des alvéoles de 1,6 ml à raison de 1,67 g (soit environ 100 mg de trimipramine) par dose puis on lyophilise. On réalise un autre essai dans lequel on place la pâte dans un cristallisoir et on met l'ensemble dans un dessicateur chauffant à 60°C pendant une nuit.

On obtient après séchage soit des lyophilisats non collants au toucher dans le premier cas soit une poudre parfaitement fluide dans le deuxième cas.

Après disssolution dans environ 20ml d'eau on obtient une solution limpide légèrement amère dans le cas de l'exemple contenant 100 mg de trimipramine base par dose et un goût insipide pour les dosages inférieurs. La solution est considérablement moins amère qu'en l'absence de cyclodextrine.

EXEMPLE COMPARATIF 1

On reproduit l'exemple 1 selon la première composition indiquée mais en l'absence de cyclodextrine c'est à dire que l'on met 6,64 g de trimipramine méthane sulfonate dans 26,9 ml d'eau et on sèche la solution obtenue. On obtient une masse sous forme de gomme que l'on ne peut remettre en solution dans l'eau.

EXEMPLE 2

On met en oeuvre la composition suivante:

```
trimipramine méthane sulfonate    :  0,44 g
ß-cyclodextrine                   :  6,20 g
eau                               :  2,50 g
isopropanol                       :  2,50 g
```

On mélange l'eau et l'isopropanol, on ajoute la cyclodextrine puis la trimipramine méthane sulfonate. On laisse sous agitation magnétique pendant une heure et demi. On évapore au rotavapor pendant 40 minutes à 60°C. La poudre obtenue est remise en solution, elle a un goût insipide quelque soit la concentration.


**Revendications**

1. Compositions pharmaceutiques solides solubles dans l'eau caractérisées en ce qu'elle sont susceptibles d'être obtenues par séchage d'une solution aqueuse contenant la β-cyclodextrine et l'imipramine, un de ses dérivés ou l'un de leurs sels selon un rapport molaire β-cyclodextrine sur imipramine, un de ses dérivés ou un de leurs sels d'au moins 2.

2. Compositions pharmaceutiques solides solubles dans l'eau caractérisées en ce qu'elles sont susceptibles d'être obtenues par séchage d'une solution aqueuse contenant de la β-cyclodextrine et du méthane sulfonate d'imipramine.

3. Composé d'inclusion solide du méthane sulfonate de trimipramine et de β-cyclodextrine.


**Patentansprüche**

1. In Wasser lösliche feste pharmazeutische Zusammensetzungen, dadurch gekennzeichnet, daß sie erhältlich sind durch Trocknen einer wäßrigen Lösung, enthaltend das ß-Cyclodextrin und das Imipramin, eines seiner Derivate oder eines ihrer Salze entsprechend einem Mol-Verhältnis von ß-Cyclodextrin zu Imipramin, einem seiner Derivate oder einem ihrer Salze von zumindest 2.

2. In Wasser lösliche feste pharmazeutische Zusammensetzungen, dadurch gekennzeichnet, daß sie erhältlich sind durch Trocknen einer wäßrigen Lösung, enthaltend das β-Cyclodextrin und Imipraminmethansulfonat.

3. Feste Einschlußverbindung von Trimipraminmethansulfonat und β-Cyclodextrin.

**Claims**

1. Water-soluble, solid pharmaceutical compositions, characterised in that they are capable of being obtained by drying an aqueous solution containing ß-cyclodextrin and imipramine, one of its derivatives or one of their salts, according to a mole ratio of ß-cyclodextrin to imipramine, one of its derivatives or one of their salts, of at least 2.

2. Water-soluble, solid pharmaceutical compositions, characterised in that they are capable of being obtained by drying an aqueous solution containing β-cyclodextrin and imipramine methanesulphonate.

3. Solid inclusion compound of trimipramine methane-sulphonate and β-cyclodextrin.